(19) Europäisches Patentamt
European Patent Office
Office européen des brevets

(11) EP 0 965 582 B1

(12) EUROPÄISCHE PATENTSCHRIFT

(45) Veröffentlichungstag und Bekanntmachung des Hinweises auf die Patenterteilung:
**10.09.2003 Patentblatt 2003/37**

(21) Anmeldenummer: **99107086.3**

(22) Anmeldetag: **12.04.1999**

(51) Int Cl.[7]: **C07C 267/00**, C07C 271/20, C08G 18/02, C08G 18/79, C08G 18/28, C08G 18/38, C08G 18/80, C08K 5/29

(54) **Carbodiimide auf der Basis von 1,3-Bis-(1-methyl-1-isocyanatoethyl)-benzol**

Carbodiimide based on 1,3-bis-(1-methyl-1-isocyanatoethyl)-benzene

Carbodiimide à base de 1,3-bis-(1-methyl-1-isocyanatoethyl)-benzène

(84) Benannte Vertragsstaaten:
**DE ES FR GB IT SE**

(30) Priorität: **14.05.1998 DE 19821666**

(43) Veröffentlichungstag der Anmeldung:
**22.12.1999 Patentblatt 1999/51**

(73) Patentinhaber: **BASF AKTIENGESELLSCHAFT**
**67056 Ludwigshafen (DE)**

(72) Erfinder:
- **Bollmann, Heinz**
  **49594 Alfhausen (DE)**
- **Häberle, Karl Dr.**
  **67346 Speyer (DE)**
- **Kokel, Nicolas Dr.**
  **13980 Alleins (FR)**
- **Thärigen, Raina**
  **49086 Osnabrück (DE)**

(56) Entgegenhaltungen:
**EP-A- 0 686 626**
**EP-A- 0 789 059**
**US-A- 5 597 942**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 965 582 B1

## Beschreibung

Die Erfindung betrifft Carbodiimide auf der Basis von 1,3-Bis-(1-methyl-1-isocyanato-ethyl)-benzol enthaltend 12 bis 40 Gew.-% Ethylenoxideinheiten [-$CH_2$-$CH_2$-O-], bezogen auf das Gewicht der Carbodiimide.

Des weiteren betrifft die Erfindung Verfahren zur Herstellung von diesen Carbodiimiden und Mischungen, enthaltend diese Carbodiimide und Verbindungen, die Esterstrukturen aufweisen, bevorzugt Polyurethane, die Esterstrukturen aufweisen.

Organische Carbodiimide sind bekannt und finden beispielsweise Verwendung als Stabilisator gegen den hydrolytischen Abbau von Estergruppen enthaltenden Verbindungen, beispielsweise Polyadditions- und Polykondensationsprodukten wie z.B. Polyurethanen. Carbodiimide können nach allgemein bekannten Verfahren hergestellt werden, beispielsweise durch Einwirkung von Katalysatoren auf Mono- oder Polyisocyanate unter Kohlendioxidabspaltung. Als Katalysatoren geeignet sind z.B. heterocyclische, Phosphor gebunden enthaltende Verbindungen z.B. Phospholine, Phospholene und Phospholidine sowie deren Oxide und Sulfide und/oder Metallcarbonyle.

Derartige Carbodiimide, ihre Herstellung und Verwendung als Stabilisatoren gegen die hydrolytische Spaltung von Kunststoffen auf Polyesterbasis werden z.B. beschrieben in DE-A 4 318 979, DE-A 4 442 724 und EP-A 460 481.

Insbesondere zur Herstellung von Elastomeren auf der Basis von Polyurethanen, beispielsweise zelligen oder mikrozelligen Polyurethanelastomeren, hat es sich als zweckmäßig erwiesen, die Carbodiimide als Stabilisatoren in die Komponente einzumischen, die Wasser als Vernetzungs- und Treibmittel enthält, um eine Reaktion von Säuregruppen mit den Carbodiimiden zu vermeiden.

Gerade die Mischbarkeit und damit die Einarbeitung von bekannten Carbodiimiden in ein solches wäßriges System gestaltet sich aufgrund der geringen Löslichkeit im allgemeinen schwierig.

DE-A 4 318 979 beschreibt die Verwendung von 1,3-Bis-(1-methyl -1-isocyanato-ethyl)-benzol zur Herstellung von Carbodiimiden und/oder oligomeren Polycarbodiimiden mit endständigen Isocyanat-, Harnstoff- und/oder Urethangruppen. Die endständigen Isocyanatgruppen der in dieser Schrift beschriebenen Carbodiimide werden mit üblichen gegenüber Isocyanaten reaktiven Verbindungen umgesetzt. Die in den Beispielen beschriebenen Carbodiimide besitzen aufgrund der Umsetzung mit Polyoxyethylenglykolen zwar einen ausreichend hydrophilen Charakter aufgrund der sehr hohen Anteile an Ethylenoxideinheiten und sind gut mit wäßrigen Komponenten mischbar, Polyurethanelastomere, die mit diesen Carbodiimiden hergestellt werden, zeigen aber im Vergleich zu Elastomeren, die ohne Stabilisator hergestellt werden, verschlechterte mechanische Eigenschaften. Verbesserungsmöglichkeiten für diese Carbodiimide ergeben sich durch den Einfluß der Stabilisatoren auf die dynamischen und mechanischen Eigenschaften insbesondere von geschäumten Polyurethanelastomeren.

Die Aufgabe der vorliegenden Erfindung bestand somit darin, Carbodiimide als Stabilisatoren gegen die hydrolytische Spaltung von Kunststoffen auf Polyesterbasis zu entwickeln, die eine optimale Einarbeitbarkeit in die Ausgangskomponenten der Kunststoffe bzw. in die Kunststoffe selbst aufweisen und zudem die dynamischen und statischen Eigenschaften der Kunststoffe, insbesondere von Polyurethanelastomeren, nicht nachteilig beeinflussen.

Diese Aufgabe konnte überraschenderweise durch Carbodiimide auf der Basis von 1,3-Bis-(1-methyl-1-isocyanato-ethyl)-benzol enthaltend 12 bis 40 Gew.-%, bevorzugt 15 bis 35 Gew.-%, Ethylenoxideinheiten [-$CH_2$-$CH_2$-O-], bezogen auf das Gewicht der Carbodiimide, gelöst werden.

Die Herstellung der erfindungsgemäßen Carbodiimide erfolgt im wesentlichen durch zwei Umsetzungsschritte. Zum einen werden (1) durch allgemein bekannte Umsetzung der Isocyanatgruppen miteinander unter Abspaltung von Kohlendioxid in Gegenwart von üblichen Katalysatoren, die für diese Umsetzung bekannt sind und eingangs beschrieben wurden, Carbodiimidstrukturen erzeugt, zum anderen werden (2) Isocyanatgruppen mit gegenüber Isocyanaten reaktiven Verbindungen zur Herstellung von Urethan- und/oder Harnstoffstrukturen umgesetzt. Die Reihenfolge dieser beiden wesentlichen Verfahrensschritte (1) und (2) ist beliebig, soweit für die jeweilige Umsetzung freie Isocyanatgruppen vorliegen.

Beispielsweise kann man die erfindungsgemäßen Carbodiimide derart erhalten, daß man 1,3-Bis-(1-methyl-1-isocyanato-ethyl)-benzol in Gegenwart von Katalysatoren unter Kohlendioxidabspaltung zu Carbodiimiden umsetzt und anschließend das Isocyanatgruppen-aufweisende Carbodiimid mit mindestens einer gegenüber Isocyanaten reaktiven Verbindung (i) umsetzt, die Ethylenoxideinheiten [-$CH_2$-$CH_2$-O-] aufweist. Das molare Verhältnis der NCO-Gruppen des Isocyanatgruppen-aufweisenden Carbodiimides zu den gegenüber den Isocyanaten reaktiven Gruppen beträgt üblicherweise 10:1 bis 0,2:1, bevorzugt 5:1 bis 0,5:1, besonders bevorzugt 1:1 bis 0,5:1, insbesondere 1:1.

Alternativ können die erfindungsgemäßen Carbodiimide dadurch erhalten werden, daß man 1,3-Bis-(1-methyl-l-isocyanatoethyl)-benzol mit mindestens einer Verbindung (i) umsetzt, die Ethylenoxideinheiten [-$CH_2$-$CH_2$-O-] aufweist, wobei das Verhältnis von eingesetzten Isocyanatgruppen zu gegenüber Isocyanaten reaktiven Gruppen mindestens 2 : 1 beträgt, und anschließend das Isocyanatgruppen aufweisende Reaktionsprodukt in Gegenwart von Katalysatoren unter Kohlendioxidfreisetzung zu Carbodiimiden umsetzt.

Nach dieser Verfahrensvariante werden zunächst bis zu 50 Gew.-%, vorzugsweise bis zu 23 Gew.-% der Isocyanatgruppen des Diisocyanates mit den gegenüber Isocyanaten reaktiven Verbindungen umgesetzt und danach

die freien Isocyanatgruppen in Gegenwart von Katalysatoren unter Kohlendioxidfreisetzung ganz oder teilweise zu Carbodiimiden und/oder oligomeren Polycarbodiimiden kondensiert. Bevorzugt führt man erst die Umsetzung zu den Carbodiimiden durch und setzt anschließend das Isocyanatgruppen-aufweisende Carbodiimid mit den gegenüber Isocyanaten reaktiven Verbindungen um.

Die Ethylenoxideinheiten in den Carbodiimiden werden durch die gegenüber Isocyanaten reaktiven Verbindungen (i) in die Carbodiimide eingebracht. Deshalb verwendet man erfindungsgemäß bei der Herstellung der erfindungsgemäßen Carbodiimide als gegenüber Isocyanaten reaktive Verbindungen (i) solche, die Ethylenoxideinheiten, bevorzugt mindestens 5, besonders bevorzugt 6 bis 200 Ethylenoxideinheiten aufweisen und üblicherweise ein zahlenmittleres Molekulargewicht von 200 bis 10000 g/mol aufweisen. Die Ethylenoxideinheiten, die als folgende Struktur dargestellt werden können [$-CH_2-CH_2-O-$], können blockweise oder verteilt in der Verbindung (i) angeordnet sein. Diese erfindungsgemäßen gegenüber Isocyanaten reaktiven Verbindungen (i) können beispielsweise hergestellt werden durch die allgemein übliche Alkoxylierung von Wasserstoff-aktiven Startersubstanzen, beispielsweise Wasser, Monoolen, Diolen, Triolen, Mono-, Di- und/oder Triaminen mit üblicherweise Molekulargewichten von 18 bis 500 g/mol, üblicherweise in Gegenwart von allgemein bekannten Katalysatoren, beispielsweise Alkalimetallhydroxiden oder Alkoholaten, mit Alkylenoxiden, beispielsweise Ethylenoxid, Propylenoxid und/oder Butylenoxid, wobei erfindungsgemäß Ethylenoxid, gegebenenfalls in Mischung mit mindestens einem weiteren Alkylenoxid, eingesetzt wird. Aufgrund dieses Herstellprozesses weisen beispielsweise diese gegenüber Isocyanaten reaktiven Verbindungen (i) als reaktive Gruppen, die mit den Isocyanaten reagieren, Hydroxylgruppen auf.

Diese erfindungsgemäßen, gegenüber Isocyanaten reaktiven Verbindungen (i) Werden in Mischung mit weiteren gegenüber Isocyanaten reaktiven Verbindungen (ii), die weniger als 5 oder keine Ethylenoxideinheiten aufweisen, bei der Herstellung der erfindungsgemäßen Carbodiimide eingesetzt. Als zusätzliche gegenüber Isocyanaten reaktiven Verbindungen kommen beispielsweise Verbindungen in Frage, die mindestens eine, üblicherweise 1 bis 3 reaktive Gruppen tragen, wobei als reaktive Gruppen z.B. Hydroxyl-, Thiöl-, primäre- und/oder sekundäre Aminogruppen in Frage kommen. Diese gegebenenfalls zusätzlich zu den erfindungsgemäßen gegenüber Isocyanaten reaktiven Verbindungen (i) einzusetzenden Substanzen weisen üblicherweise Molekulargewichte von 32 bis 500 g/mol auf. Beispielsweise kommen Verbindungen in Frage, die durch ihre Reaktion mit Isocyanaten Urethan- und/oder Harnstoffgruppen erzeugen. Beispielsweise können aromatische, aliphatische und/oder araliphatische Verbindungen mit 1 bis 20 Kohlenstoffatomen verwendet werden, die Hydroxyl- und/oder Amingruppen als gegenüber Isocyanaten reaktive Gruppen enthalten. Beispielsweise können als Verbindungen, die gegenüber Isocyanaten reaktive Gruppen aufweisen, organische Verbindungen mit mindestens einer Hydroxylgruppe, mit mindestens einer Amingruppe und/oder mindestens einer Hydroxylgruppe und mindestens einer Amingruppe eingesetzt werden. Beispielsweise können die in der DE-A 4 318 979 genannten Alkohole verwendet werden. Überdies können aromatische, araliphatische und/oder aliphatische Polyole mit 2 bis 20 Kohlenstoffatomen, verwendet werden. Z.B. können genannt werden: Ethandiol-1,2, Propandiol-1,3, Propandiol-1,2, Butandiol-1,4, -2,4, und/oder -2,3, Pentandiol-1,5, Hexandiol-1,6, Heptandiol-1,7, Octandiol-1,8, Dekandiol-1,10, Neopentylglykol, 2-Methylpropandiol-1,3, 2- und 3-Methylpentandiol-1,5, die Isomeren des Bis(hydroxy-methyl- oder -ethyl)benzols, Hydroxyalkylether von Dihydroxybenzolen, Trimethylolpropan, Glycerin, Pentaerythrit und/oder Zucker mit beispielsweise 4, 5 oder 6 Hydroxylgruppen.

Als Amine sind Amine mit mindestens einer primären und/oder sekundären Amingruppe zu verwenden. Beispielhaft sind zu nennen: Amine des Molekulargewichtsbereiches von 31 bis 500 g/mol, vorzugsweise von 60 bis 300 g/mol, welche mindestens eine primäre oder sekundäre Aminogruppe enthalten. Weitere Beispiele sind Diamine wie Diaminoethan, Diaminopropan, Diaminobutan, Diaminopentan, Diaminohexan, Piperazin, 2,5-Dimethylpiperazin, 1-Amino-3,3,5-trimethyl-5-aminomethyl-cyclohexan (Isophorondiamin, IPDA), 4,4'-Diaminodicyclohexylmethan, 1,4-Diaminocyclohexan, Aminoethylethanolamin, Hydrazin, Hydrazinhydrat und/oder Triamine wie Diethylentriamin, und/oder 1,8-Diamino-4-aminomethyloctan.

Weiterhin können Amine verwendet werden, die sich von den genannten Aminen dadurch ableiten, daß eine oder mehrere primäre Amingruppen durch weitere Substituenten wie z.B. Alkylgruppen zu sekundären Amingruppen substituiert werden. Des weiteren können auch Verbindungen, die sowohl mindestens eine Hydroxylgruppe als auch mindestens eine Amingruppe aufweisen, eingesetzt werden, beispielsweise Ethanolamin, Propanolamin, Isopropanolamin, Aminoethylethanolamin oder davon abgeleitete N-Alkylamine.

Geeignete Alkohole und Amine sind z.B. in der DE-A 4 318 979, Seite 4, Zeilen 29 bis 33 genannt.

Bevorzugt werden die erfindungsgemäßen gegenüber Isocyanaten reaktiven Verbindungen (i) zusammen mit monofunktionellen Verbindungen eingesetzt, um das Molekulargewicht der erfindungsgemäßen Carbodiimide zu regulieren, insbesondere wenn die Diisocyanate in einem ersten Schritt zu den Carbodiimiden umgesetzt werden und anschließend die Reaktion der Isocyanatgruppen-enthaltenden Carbodiimide mit den gegenüber Isocyanaten reaktiven Verbindungen erfolgt. Als monofunktionelle gegenüber Isocyanaten reaktive Verbindungen können beispielsweise Amine und bevorzugt Alkohole verwendet werden. Geeignete Amine, z.B. primäre oder vorzugsweise sekundäre Amine, besitzen vorteilhafterweise 1 bis 12 C-Atome, vorzugsweise 2 bis 8 C-Atome. Beispielhaft genannt seien Methyl-, Ethyl-, Propyl-, Butyl-, Pentyl-, Hexyl-, 2-Ethylhexyl-, Octyl-, Decyl-, Diethyl-, Dipropyl-, Dibutyl-, Methylbutyl-, Ethylbutyl- und Ethylhexylamin sowie Cyclohexyl- und Benzylamin. Zur Absättigung der Isocyanatgruppen finden jedoch vorzugsweise Alkohole, z.B. primäre oder sekundäre Alkohole mit 1 bis 18 C-Atomen, vorzugsweise 2 bis 8 C-Atomen Verwendung. Als primäre oder sekundäre Alkohole seien beispielhaft genannt: Methanol, Ethanol, n-Propanol, Isopropanol, n-Butanol, sekundärButanol, n-Pentanol, technische Pentanolmischungen, n-Hexanol, technische Hexanolgemische, 2-Ethylhexanol, Octanol, 2-Ethyloctanol, Dekanol und Dodekanol sowie Cyclohexanol und Benzylalkohol.

Weiterhin bevorzugt sind Alkoxylierungsprodukte wie die Verbindungen (i), jedoch mit der Maßgabe, daß sie weniger als 5 EO-Einheiten enthalten. Solche Alkoxylierungsprodukte tragen nicht zur Hydrophilie der erfindungsgemäßen Carboddimide bei; sie sind also für die Berechnung des EO-Gehaltes nicht in Betracht zu ziehen. Beispielsweise zu nennen sind hier Monoalkylether von Mono-, Di-, Tri- und Tetraethylenglykol wie Diethylenglykolmonobutylether oder Diethylenglykolmonoethylether.

Man verwendet als gegenüber Isocyanaten reaktive Verbindungen zur Herstellung der erfindungsgemäßen Carbodiimide eine Mischung, die enthält:

(i) gegenüber Isocyanaten reaktive Verbindungen, die mindestens 5 Ethylenoxideinheiten aufweisen und eingangs bereits beschrieben wurden und

(ii) gegenüber Isocyanaten reaktive Verbindungen, bevorzugt mindestens einen Monoalkohol und/oder ein Monoamin, besonders bevorzugt mindestens einen Monoalkohol, die keine oder weniger als 5 Ethylenoxideinheiten aufweisen.

Bei der Umsetzung der Isocyanatgruppen mit den gegenüber Isocyanaten reaktiven Verbindungen wird die gegenüber Isocyanaten reaktive Verbindung (i) bevorzugt im Unterschuß zu den Isocyanatgruppen, besonders bevorzugt mit einem Verhältnis von NCO-Gruppen zu den gegenüber Isocyanaten reaktiven Gruppen von 1:0,1 bis 1:0,9 eingesetzt. Verbleibende Isocyanatgruppen..werden bevorzugt durch die Monoole umgesetzt. Es können jedoch auch Isocyanatgruppen des Carbodiimids erhalten bleiben. Das molare Verhältnis von (i) : (ii) beträgt bevorzugt 0,1 : 1 bis 9:1.

In jedem Falle muß bei der Herstellung der erfindungsgemäßen Carbodiimide durch die Art der gegenüber Isocyanaten reaktiven Verbindungen, beispielsweise durch das Verhältnis von (i) zu (ii), sichergestellt sein, daß in den erfindungsgemäßen Verfahrensprodukten, den Carbodiimiden, 12 bis 40 Gew.-% Ethylenoxideinheiten, bezogen auf das Gesamtgewicht der Carbodiimide, enthalten sind.

Der Verfahrensschritt (1) zur Herstellung der erfindungsgemäßen Carbodiimide durch Umsetzung von Diisocyanaten kann bei erhöhten Temperaturen, z.B. bei Temperaturen von 50 bis 200°C, vorzugsweise von 150 bis 185°C, zweckmäßigerweise in Gegenwart von Katalysatoren unter Kohlendioxidabspaltung kondensiert werden. Hierfür geeignete Verfahren werden beispielsweise beschrieben in der GB-A-1 083 410, der DE-A 1 130 594 und der DE-A-11 56 401.

Als Katalysatoren vorzüglich bewährt haben sich z.B. Phosphorverbindungen, die vorzugsweise ausgewählt werden aus der Gruppe der Phospholene, Phospholenoxide, Phospholidine und Phospholinoxide. Wenn die Reaktionsmischung den gewünschten Gehalt an NCO-Gruppen besitzt, wird die Polycarbodiimidbildung üblicherweise beendet. Hierzu können die Katalysatoren unter vermindertem Druck abdestilliert oder durch Zusatz eines Desaktivators, wie z.B. Phosphortrichlorid, desaktiviert werden. Die Polycarbodiimidherstellung kann ferner in Abwesenheit oder Gegenwart von unter den Reaktionsbedingungen inerten Lösungsmitteln durchgeführt werden.

Durch geeignete Wahl der Reaktionsbedingungen wie z.B. der Reaktionstemperatur, der Katalysatorart und der Katalysatormenge sowie der Reaktionszeit kann der Fachmann in der üblichen Weise den Kondensationsgrad einstellen. Der Verlauf der Reaktion kann am einfachsten durch Bestimmung des NCO-Gehaltes verfolgt werden. Auch andere Parameter wie z.B. Viskositätsanstieg, Farbvertiefung oder $CO_2$-Entwicklung kann man für die Verfolgung des Ablaufs und die Steuerung der Reaktion heranziehen.

Als Diisocyanat wird zur Herstellung der erfindungsgemäßen Carbodiimide 1,3-Bis-(1-methyl-1-isocyanatoethyl)-benzol, im Folgenden auch als TMXDI bezeichnet, eingesetzt. Das TMXDI kann in Mischungen mit weiteren, allgemein üblichen Isocyanaten verwendet werden, beispielsweise Hexamethylendiisocyanat, 1-Isocyanato-3,3,5-trimethyl-5-isocyanatomethyl-cyclohexan (Isophorondiisocyanat), Di(cyclohexyl)methandiisocyanat, Trimethylhexamethylendiisocyanat, Dodecandiisocyanat, Octandiisocyanat und/oder Cyclohexan-1,4-diisocyanat. In diesem Fall werden bevorzugt mindestens 30 mol% an TMXDI mitverwendet. Die erfindungsgemäßen Carbodiimide weisen somit mindestens eine der folgenden Struktureinheiten auf, die die Carbodiimidstruktur des erfindungsgemäßen Diisocyanats darstellt:

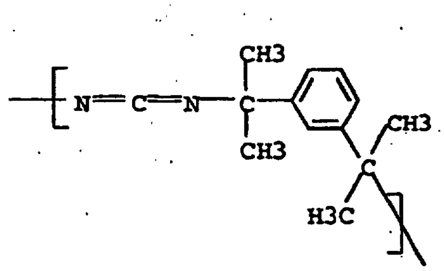

Beispielsweise können die erfindungsgemäßen Carbodiimide folgende Struktur aufweisen:

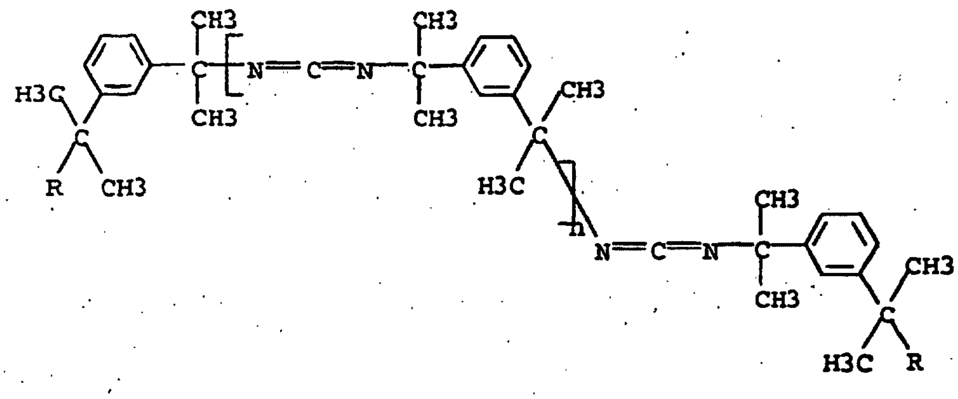

(I)

in der

R gleich oder verschieden, beispielsweise -NHCONHR$^1$- oder -NHCOOR$^1$-Reste, wobei sich R$^1$ und R$^2$ aus den gegenüber Isocyanaten reaktiven Verbindungen, die bereits eingangs in Form der Verbindungen (i) und (ii) beispielhaft beschrieben wurden, ergeben und die die abgebildete Struktur mit weiteren Carbodiimidstrukturen verbinden können,

n beispielsweise 0, 1, 2, 3, 4, 5, 6, 7, 8, 9, 10

bedeuten können, wobei n wie angegeben ganzzahlig oder - im statistischen Mittel - gebrochen sein kann.

Die erfindungsgemäßen Carbodiimide enthaltend mindestens eine, bevorzugt eine bis zwanzig Carbodiimidstrukturen, besonders bevorzugt beträgt der mittlere Kondensationsgrad (Zahlenmittelwert), d.h. die mittlere Anzahl an Carbodiimidstrukturen in den erfindungsgemäßen Carbodiimiden eins bis zehn. Des weiteren enthalten die erfindungsgemäßen Verbindungen Urethan- und/oder Harnstoffstrukturen, die durch die Umsetzung von Isocyanatgruppen der bei der Herstellung eingesetzten Diisocyanate mit gegenüber Isocyanaten reaktiven Verbindungen entstehen.

Die Carbodiimidstrukturen der erfindungsgemäßen Verbindungen sind an nicht-aromatische Kohlenwasserstoffe gebunden. Dies bietet den wesentlichen Vorteil, daß bei einer Spaltung der Carbodiimide keine aromatischen Amine freigesetzt werden und somit die erfindungsgemäßen Carbodiimide toxikologisch wesentlich weniger bedenklich sind als die beispielsweise in EP-A 460 481 beschriebenen Carbodiimide.

Aus den erfindungsgemäßen Carbodiimiden entstehen bei der Reaktion mit Carbonsäuren und/oder carboxylgruppenhaltigen Verbindungen im Falle von Carbodiimiden auf der Basis von TMXDI araliphatische Isocyanate mit einer im Vergleich zu aromatischen Isocyanaten geringen Reaktivität. Die gebildeten araliphatischen Isocyanate beeinflussen daher beispielsweise die Kennzahl einer Polyadditionsreaktion bei einer Urethanbildung praktisch nicht. Folglich sind die Molekulargewichte der gebildeten Polyurethane und damit verbunden ihre mechanischen Eigenschaften konstant und sehr gut reproduzierbar.

Die erfindungsgemäßen Carbodiimide zeigen eine mit den technisch genutzten aromatischen Carbodiimiden und aromatischen Polycarbodiimiden zumindest vergleichbare hohe Hydrolyseschutzwirkung und Lichtbeständigkeit und können unter Beachtung der Arbeitsschutzvorschriften problemlos in die Estergruppen enthaltenden Polykondensations- und Polyadditionsprodukte eingebracht werden. Die Carbodiimide sind mit den Estergruppen enthaltenden Polyadditions- und Polykondensationsprodukten, insbesondere mit Polyesterurethankautschuken, gut verträglich und

auch problemlos mit diesen Materialien in der Schmelze homogen mischbar.

Die erfindungsgemäßen Monocarbodiimide und/oder oligomeren Polycarbodiimide eignen sich hervorragend als Akzeptor für Carboxylverbindungen und finden daher vorzugsweise Verwendung als Stabilisatoren gegen den hydrolytischen Abbau von Estergruppen enthaltenden Verbindungen, beispielsweise Estergruppen enthaltende Polymere, z.B. Polykondensationsprodukte wie beispielsweise thermoplastische Polyester wie Polyethylen- und -butylenterephthalat, Polyetherester, Polyamide, Polyesteramide, Polycaprolactone sowie ungesättigte Polyesterharze und Polyesterester wie z.B. Blockcopolymere aus Polyethylen- oder butylenterephthalat und Polycaprolacton und Polyadditionsprodukte, z.B. Polyurethane, Polyharnstoffe und Polyurethan-Polyharnstoff-Elastomere, die Estergruppen enthalten. Diese Estergruppen enthaltenden Verbindungen sind allgemein bekannt. Ihre Ausgangssubstanzen, Herstellverfahren, Strukturen und Eigenschaften sind in der Standardliteratur vielfältig beschrieben. Aufgrund der guten Löslichkeit in den Aufbaukomponenten zur Herstellung von Polyurethanen und der guten Verträglichkeit mit den gebildeten Polyurethanen eignen sich die erfindungsgemäßen (Poly)carbodiimide insbesondere als Stabilisatoren gegen den hydrolytischen Abbau von Polyurethanen, vorzugsweise kompakten oder zelligen Polyurethan-Elastomeren und insbesondere thermoplastischen Polyurethanen sowie zelligen oder kompakten Elastomeren.

Weisen die erfindungsgemäßen Carbodiimide endständige Isocyanatgruppen auf, beispielsweise wenn ein Isocyanatgruppen aufweisendes Carbodiimid mit einem Unterschuß an gegenüber Isocyanaten reaktiven Gruppen eingesetzt wird, können die Carbodiimide bei der Herstellung der Polyadditionsprodukte durch Umsetzung von Isocyanaten mit gegenüber Isocyanaten reaktiven Verbindungen eingesetzt werden. Des weiteren können die erfindungsgemäßen Carbodiimide als Stabilisatoren von Kunststoffen enthaltend übliche Polyamide, Polyoxymethylenhomo- oder copolymerisate, die dem Fachmann an sich bekannt und in der Literatur beschrieben sind, verwendet werden.

Des weiteren können die erfindungsgemäßen Carbondiimide als Vernetzer in oder für wäßrige Latices verwendet werden. Wäßrige Latices sind beispielsweise in DE-A 3 512 918, EP-A 548815, EP-A 582 983 und EP-A 686 626 beschrieben.

Die Konzentration der erfindungsgemäßen Carbodiimide in den zu stabilisierenden Estergruppen enthaltenden Polykondensationsoder Polyadditionsprodukte beträgt im allgemeinen 0,05 bis 10 Gew.-%, vorzugsweise 0.1 bis 5 Gew.-%, bezogen auf das Gesamtgewicht der Mischung. In Einzelfällen kann, je nach der' Beanspruchung des Kunststoffs durch Hydrolyse, die Konzentration auch höher sein.

Die erfindungsgemäß verwendbaren Carbodiimide können nach verschiedenen Methoden in die zu stabilisierenden Estergruppen enthaltenden Produkte eingebracht werden. Beispielsweise können die erfindungsgemäßen Carbodiimide mit einer der Aufbaukomponenten zur Herstellung der Polyadditionsprodukte, z.B. den Polyisocyanaten oder/und Polyhydroxylverbindungen zur Herstellung von Polyurethanen, gemischt werden oder die Carbodiimide können der Reaktionsmischung zur Herstellung der Polyurethane zudosiert werden. Nach einer anderen Verfahrensweise können die erfindungsgemäßen Carbodiimide der Schmelze der ausreagierten Polyadditions- oder Polykondensationsprodukte einverleibt werden. Es ist jedoch auch möglich, Granulate der Polyadditions- oder Polykondensationsprodukte mit den erfindungsgemäßen Carbodiimiden zu beschichten oder mit den pulverisierten, pelletierten oder granulierten erfindungsgemäßen Carbodiimiden zu mischen und bei einer nachfolgenden Herstellung von Formkörpern durch Schmelzextrusion in die Kunststoffmassen einzubringen. Zur Herstellung von Polyurethan-Gießelastomeren und TPU auf Polyesterbasis werden nach einer bevorzugten Ausführungsform zunächst die carboxylgruppenhaltigen Polyester-polyole zur Reduzierung der Säuregehalte mit den erfindungsgemäßen Carbodiimiden behandelt und danach diese, gegebenenfalls unter Zugabe von weiteren Mengen an Carbodiimiden, mit Polyisocyanaten, gegebenenfalls in Gegenwart zusätzlicher Hilfsmittel und Additive, zur Reaktion gebracht. Weiter können die erfindungsgemäßen Carbodiimide über die Isocyanat-Komponente in das Polyurethan eingebracht werden. Besonders zum Tragen kommen die erfindungsgemäßen Carbodiimide jedoch dann, wenn sie während der üblichen Konfektionierung in das estergruppenhaltige Polymer eingebracht werden.

Besonders bevorzugt werden die erfindungsgemäßen Carbodiimide bei der Herstellung von Polyurethanen, z.B. zelligen, beispielsweise mikrozelligen Polyurethanen, insbesondere Polyurethanelastomeren, eingesetzt. Die Herstellung dieser Polyurethane, insbesondere Polyurethanelastomere kann durch bekannte Umsetzung von üblichen Ausgangskomponenten, d.h. Isocyanaten, gegenüber Isocyanaten reaktiven Verbindungen, Treibmitteln, bevorzugt Wasser und gegebenenfalls Katalysatoren, Hilfs- und/oder Zusatzstoffen in Gegenwart der erfindungsgemäßen Carbodiimide erfolgen. Dabei gibt man bevorzugt die erfindungsgemäßen Carbodiimide zu der Komponente, die das Treibmittel, bevorzugt Wasser enthält.

Als Ausgangskomponenten zur Herstellung der Polyurethane, insbesondere der Polyurethanelastomere können beispielsweise folgende Verbindungen Verwendung finden:

Als Isocyanate können allgemein übliche Isocyanate, bevorzugt organische Diisocyanate eingesetzt werden. Im einzelnen seien beispielhaft genannt: Alkyleridiisocyanate mit 4 bis 12 Kohlenstoffatomen im Alkylenrest, wie 1,12-Dodecandiisocyanat, 2-Ethyltetramethylendiisocyanat-1,4, 2-Methylpentamethylendiisocyanat-1,5. Tetramethylendiisocyanat-1,4, Lysinesterdiisocyanate (LDI) und/oder Hexamethylendiisocyanat-1,6 (HDI); cycloaliphatische Diisocyanate wie Cyclohexan-1,3- und 1,4-diisocyanat sowie beliebige Gemische dieser Isomeren, 2,4- und 2,6-Hexahydrotoluylendiisocyanat sowie die entsprechenden Isomerengemische, 4,4'-, 2,2'- und 2, 4'-Dicyclohexylmethandiisocyanat sowie die entsprechenden Isomerengemische und/oder 1-Isocyanatc-3,3,5-trimethyl-5-isocyanatomethylcyclohexan (IPDI). Des weiteren kommen aromatische Di- und Polyisocyanate, wie z.B. 2,4- und 2,6-Toluylendiisocyanat und die entsprechenden Isomerengemische, 4,4'-, 2,4'- und 2,2'-Diphenylmethandiisocyanat und die entsprechenden Isomerengemische, Naphthylendiisocyanat, Polyphenylpolymethylen-polyisocyanate, Mischungen aus 4,4'-, 2,4'- und 2,2'-Diphenylmethandiisocyanaten und Polyphenylpolymethylen-polyisocyanaten (Roh-MDI) und Mischungen aus Roh-MDI und Toluylendiisocyanaten in Betracht. Außerdem können Ester-, Harnstoff-, Allophanat-, Isocyanaturat-, Biuret-, Uretdion- und/oder Urethangruppen enthaltende Di- und/oder Polyisocyanate in dem erfindungsgemäßen Verfahren eingesetzt werden.

Die Isocyanate können allein oder in Mischungen verwendet werden.

Als gegenüber Isocyanaten reaktive Verbindungen können allgemein bekannte Verbindungen mit einem üblichen Molekulargewicht von 60 bis 10000, die mindestens eine, bevorzugt 2 bis 6 gegenüber Isocyanaten reaktive Gruppen, beispielsweise Hydroxyl-, Thio- und/oder Aminogruppen, enthalten, eingesetzt werden. Bewährt haben sich z.B. Polyole ausgewählt aus der Gruppe der Polyetherpolyole, Polyesterpolyole, Polythioether-polyole, hydroxylgruppenhaltigen Polyacetale und hydroxylgruppenhaltigen aliphatischen Polycarbonate oder Mischungen aus mindestens zwei der genannten Polyole'. Vorzugsweise Anwendung finden Polyesterpolyole und/oder Polyetherpolyole, bevorzugt Polyether- und Polyesterpolyole.

Als Polyetherpolyole seien beispielhaft genannt: Polytetrahydrofuran, Polyetherpolyole, die hergestellt werden durch übliche Anlagerung von Alkylenoxiden an Startermoleküle, wobei bevorzugt zum Abschluß Ethylenoxid angelagert wird, womit durch die endständigen Ethylenoxideinheiten primäre Hydroxylgruppen in den Polyetherpolyolen erzeugt werden. Die Polyetherpolyalkohole weisen bevorzugt ein Molekulargewicht von 450 bis 8000 auf. Geeignete Polyesterpolyole können beispielsweise aus organischen Dicarbonsäuren mit 2 bis 12 Kohlenstoffatomen, vorzugsweise aliphatischen Dicarbonsäuren mit 4 bis 6 Kohlenstoffatomen, und mehrwertigen Alkoholen, vorzugsweise Diolen, mit 2 bis 12 Kohlenstoffatomen, vorzugsweise 2 bis 6 Kohlenstoffatomen hergestellt werden. Als Dicarbonsäuren kommen beispielsweise in Betracht: Bernsteinsäure, Glutarsäure, Adipinsäure, Korksäure, Azelainsäure, Sebacinsäure, Decandicarbonsäure, Maleinsäure, Fumarsäure, Phthalsäure, Isophthalsäure und Terephthalsäure. Die Dicarbonsäuren können dabei sowohl einzeln als auch im Gemisch untereinander verwendet werden. Anstelle der freien Dicarbonsäuren können auch die entsprechenden Dicarbonsäurederivate, wie z.B. Dicarbonsäureester von Alkoholen mit 1 bis 4 Kohlenstoffatomen oder Dicarbonsäureanhydride eingesetzt werden. Vorzugsweise verwendet werden Dicarbonsäuregemische aus Bernstein-, Glutar- und Adipinsäure in Mengenverhältnissen von beispielsweise 20 bis 35 : 35 bis 50 : 20 bis 32 Gew.-Teilen, und insbesondere Adipinsäure. Beispiele für zwei-und mehrwertige Alkohole, insbesondere Diole sind: Ethandiol, Diethylenglykol, 1,2- bzw. 1,3-Propandiol, Dipropylenglykol, 1,4-Butandiol, 1,5-Pentandiol, 1,6-Hexandiol, 1,10-Decandiol, Neopentylglykol, Glycerin und Trimethylolpropan. Vorzugsweise verwendet werden Ethandiol, Diethylenglykol, 1,4-Butandiol, 1,5-Pentandiol, 1,6-Hexandiol, Neopentylglykol oder Mischungen aus mindestens zwei der genannten Diole, insbesondere Mischungen aus 1,4-Butandiol, 1,5-Pentandiol und/oder 1,6-Hexandiol. Eingesetzt werden können ferner Polyester-polyole aus Lactonen, z.B. ε-Caprolacton oder Hydroxycarbonsäuren, z.B. ω-Hydroxycapronsäure.

Die Polyesterpolyole besitzen vorzugsweise eine Funktionalität von 2 bis 4, insbesondere 2 bis 3, und ein Molekulargewicht von 480 bis 4000, vorzugsweise 600 bis 3000 und insbesondere 600 bis 2500.

Als gegenüber Isocyanaten reaktive Verbindungen können gegebenenfalls Diole und/oder Triole mit Molekulargewichten von 62 bis 400 als Kettenverlängerungs- und/oder Vernetzungsmittel bei dem erfindungsgemäßen Verfahren eingesetzt werden. Zur Modifizierung der mechanischen Eigenschaften, z.B. der Härte; kann sich jedoch der Zusatz von Kettenverlängerungsmitteln, Vernetzungsmitteln oder gegebenenfalls auch Gemischen davon als vorteilhaft erweisen. Die Kettenverlängerungs- und/oder Vernetzungsmittel weisen vorzugsweise ein Molekulargewicht von 62 bis 300 auf. In Betracht kommen beispielsweise aliphatische, cycloaliphatische und/ oder araliphatische Diole mit 2 bis 14, vorzugsweise 4 bis 10 Kohlenstoffatomen, wie z.B. Ethylenglykol, Propandiol-1,2 und 1,3, Decandiol-1,10, 1,2-, 1,3- und 1,4-Dihydroxycyclohexan, Diethylenglykol, Dipropylenglykol und vorzugsweise Butandiol-1,4, Hexandiol-1,6 und Bis-(2-hydroxy-ethyl)-hydrochinon, Triole, wie 1,2,4-, 1,3,5-Trihydroxy-cyclohexan, Glycerin und Trimethylolpropan und niedermolekulare hydroxylgruppenhaltige Polyalkylenoxide auf Basis Ethylen- und/oder 1,2-Propylenoxid und den vorgenannten Diolen und/oder Triolen als Startermoleküle.

Sofern zur Herstellung der Polyurethanschaumstoffe Kettenverlängerungsmittel, Vernetzungsmittel, beispielsweise Wasser, oder Mischungen davon Anwendung finden, kommen diese zweckmäßigerweise in einer Menge von 0 bis 20 Gew.-%, vorzugsweise von 2 bis 8 Gew.-%, bezogen auf das Gewicht der insgesamt eingesetzten gegenüber Isocyanaten reaktiven Verbindungen, zum Einsatz.

Als Treibmittel können chemisch und/oder physikalisch wirkende Treibmittel verwendet werden. Geeignet sind als solche physikalisch wirkenden Treibmittel Flüssigkeiten, welche gegenüber den gegebenenfalls modifizierten Polyisocyanaten inert sind und Siedepunkte unter 100°C, vorzugsweise unter 50°C, insbesondere zwischen -50°C und 30°C bei Atmosphärendruck aufweisen, so daß sie unter dem Einfluß der exothermen Polyadditionsreaktion verdampfen. Beispiele derartiger, vorzugsweise verwendbarer Flüssigkeiten sind Alkane, wie Heptan, Hexan, n- und iso-Pentan, vorzugsweise technische Gemische aus n- und iso-Pentanen, n- und iso-Butan und Propan, Cycloalkane, wie Cyclopentan und/oder Cyclohexan, Ether, wie Furan, Dimethylether und Diethylether, Ketone, wie Aceton und Methylethylketon, Carbonsäurealkylester, wie Methylformiat, Dimethyloxalat und Ethylacetat und halogenierte Kohlenwasserstoffe wie beispielsweise übliche Fluorkohlenwasserstoffe und/oder Chlorkohlenwasserstoffe, wie beispielsweise Dichlormethan. Auch Gemische dieser niedrigsiedenden Flüssigkeiten untereinander und/oder mit anderen substituierten oder unsubstituierten Kohlenwasserstoffen können verwendet werden. Als chemisch wirkendes Treibmittel wird bevorzugt Wasser eingesetzt, das mit Isocyanatgruppen unter Bildung von Kohlendioxid reagiert. Besonders bevorzugt wird Wasser, gegebenenfalls in Kombination mit weiteren Treibmitteln, eingesetzt. Geeignet sind ferner organische Carbonsäuren, wie z.B. Ameisensäure, Essigsäure, Oxalsäure, Ricinolsäure und carboxylgruppenhaltige Verbindungen.

Die eingesetzte Menge des physikalischen wirkenden Treibmittels liegt bevorzugt bei 0,5 bis 25 Gew.-%, besonders bevorzugt 3 bis 15 Gew.-%, jeweils bezogen auf das Gewicht der insgesamt eingesetzten gegenüber Isocyanaten reaktiven Verbindungen.

Das Wasser wird vorzugsweise als Vernetzerkomponente zu einem Prepolymeren zugesetzt.

Als Katalysatoren können allgemein bekannte Verbindungen eingesetzt werden, die die Reaktion der Isocyanatgruppen mit den gegenüber Isocyanaten reaktiven Gruppen stark beschleunigen, wobei vorzugsweise ein Gesamtkatalysatorgehalt von 0,001 bis 15 Gew.-%, insbesondere 0,05 bis 6 Gew.-%, bezogen auf das Gewicht der insgesamt eingesetzten gegenüber Isocyanaten reaktiven Verbindungen, verwendet wird, beispielsweise folgenden Verbindungen: Triethylamin, Tributylamin, Dimethylbenzylamin, Dicyclohexylmethylamin, Dimethylcyclohexylamin, N,N,N',N'-Tetramethyl-diamino-diethylether, Bis-(dimethylaminopropyl)-harnstoff, N-Methyl- bzw. N-Ethylmorpholin, N-Cyclohexylmorpholin, N,N,N',N'-Tetramethylethylendiamin, N,N,N',N'-Tetramethylbutandiamin, N,N,N',N'-Tetramethylhexandiamin-1,6, Pentamethyldiethylentriamin, Dimethylpiperazin, N-Dimethylaminoethylpiperidin, 1,2-Dimethylimidazol, 1-Azabicyclo-(2,2,0)-octan, 1,4-Diazabicyclo-(2,2,2)-octan(Dabco) und Alkanolaminverbindungen, wie Triethanolamin, Triisopropanolamin, N-Methyl- und N-Ethyl-diethanolamin, Dimethylaminoethanol, 2-(N,N-Dimethylaminoethoxy) ethanol, N,N',N''-Tris-(dialkylaminoalkyl)hexahydrotriazine, z.B. N,N',N''-Tris-(dimethylaminopropyl)-s-hexahydrotriazin, Triethylendiamin, Pentamethylendiethylentriamin und/oder Bis(dimethylamino)ether, Eisen(II)-chlorid, Zinkchlorid, Bleioctoat, Zinndioctoat, Zinndiethylhexoat, Dibutylzinndilaurat und/oder Dibutyldilaurylzinnmercaptid, 2,3-Dimethyl-3,4,5,6-tetrahydropyrimidin, Tetraalkylammoniumhydroxide, wie Tetramethylammoniumhydroxid, Alkalihydroxide, wie Natriumhydroxid und Alkalialkoholate, wie Natriummethylat und Kaliumisopropylat, sowie Alkalisalze von langkettigen Fettsäuren mit 10 bis 20 C-Atomen und gegebenenfalls seitenständigen OH-Gruppen.

Als Hilfs- und/oder Zusatzstoffe seien beispielsweise genannt: oberflächenaktive Substanzen, Schaumstabilisatoren, Zellregler, Füllstoffe, Farbstoffe, Pigmente, Flammschutzmittel, fungistatische und bakteriostatisch wirkende Substanzen.

Zur Herstellung der der Polyurethane, insbesondere der Polyurethanelastomere, werden die Isocyanate und die gegenüber Isocyanaten reaktiven Verbindungen in Gegenwart der erfindungsgemäßen Carbodiimide solchen Mengen zur Umsetzung gebracht, daß das Äquivalenzverhältnis von NCO-Gruppen der Isocyanate zur Summe der reaktiven Wasserstoffatome der gegenüber Isocyanaten reaktiven Verbindungen 0,85 bis 1,25 : 1, vorzugsweise 0,95 bis 1,15 : 1 und insbesondere 1 bis 1,05 : 1, beträgt. Falls die Polyurethane zumindest teilweise Isocyanuratgruppen gebunden enthalten, wird üblicherweise ein Verhältnis von NCO-Gruppen zur Summe der reaktiven Wasserstoffatome von 1,5 bis 60 : 1, vorzugsweise 1,5 bis 8:1, angewandt.

Als gegenüber Isocyanaten reaktive Verbindungen bzw. Gruppen sind per Definition dieser Schrift keine Isocyanate oder Isocyanatgruppen zu verstehen. Die erfindungsgemäßen Carbodiimide sind, soweit sie Isocyanatgruppen oder gegenüber Isocyanaten reaktive Gruppen enthalten, bei der obigen Rechnung mit einzubeziehen.

Die Polyurethane werden vorteilhafterweise nach dem one shot-Verfahren oder dem Prepolymerverfahren, beispielsweise mit Hilfe der Hochdruck- oder Niederdruck-Technik in offenen oder geschlossenen Formwerkzeugen, beispielsweise metallischen Formwerkzeugen, hergestellt. Üblich ist auch das kontinuierliche Auftragen des Reaktionsgemisches auf geeigneten Bandstraßen zur Erzeugung von Paneelen.

Als besonders vorteilhaft hat es sich erwiesen, nach dem Zweikomponentenverfahren zu arbeiten und die gegenüber Isocyanaten reaktiven Verbindungen, das oder die Treibmittel und gegebenenfalls die Katalysatoren, Hilfs- und/oder Zusatzstoffe in der Komponente (A) zu vereinigen und als Komponente (B) die Isocyanate oder Mischungen aus den Isocyanaten und gegebenenfalls Treibmittel zu verwenden.

Die erfindungsgemäßen Carbodiimide werden dabei bevorzugt in die Komponente A, besonders bevorzugt in die gegebenenfalls darin enthaltenen Ketten- und/oder Vernetzungsmittel eingearbeitet.

Gerade bei dem Einsatz als Stabilisatoren in Polyurethanelastomereh zeigen die erfindungsgemäßen Carbodiimide ihre deutlichen Vorteile gegenüber den bekannten. Insbesondere haben die erfindungsgemäßen Stabilisatoren keinen negativen Einfluß auf die statischen und dynamischen Eigenschaften der Polyurethanelastomere. Die erfindungsgemäßen Carbodiimide sind aufgrund ihres Ethylenoxidgehaltes sehr gut in wäßrige Komponenten einzuarbeiten, zudem zeigen die erfindungsgemäß hergestellten Polyurethanelastomere im Vergleich zu Elastomeren, die mit bekannten, z.B. aus DE-A 43 18 979, Carbodiimiden hergestellt werden, deutlich verbesserte Eigenschaften.

Neben der Wirksamkeit als Stabilisator gegen den hydrolytischen Abbau von Estergruppen enthaltenden Polyadditions- oder Polykondensationsprodukten oder zur Entsäuerung von Polyesterolen, welche zur Herstellung von polyesterhaltigen Kunststoffen, insbesondere Polyurethankautschuken, verwendet werden können, eignen sich die Carbodiimide z.B. auch zum Abbruch von Veresterungsreaktionen bei der Herstellung von Polyestern, wenn der gewünschte Polykondensationsgrad erreicht ist.

Beispiele

750 Gew.-Teile (3,1 mol) 1,3-Bis-(1-methyl-1-isocyanato-ethyl)-benzol mit einem NCO-Gehalt von 34.4 Gew.-% wurden in Gegenwart von 1,5 Gew.-Teilen, bezogen auf das Isocyanat, 1-Methyl-2-phospholen-1-oxid lösungsmittelfrei auf 180°C erhitzt und bei dieser Temperatur unter mäßiger Kohlendioxidentwicklung kondensiert. Nach Erreichen eines NCO-Gehalts der Reaktionsmischung von ca. 11 Gew.-%, wurde der zugesetzte Katalysator und Reste von nicht umgesetztem 1,3-Bis-(1-methyl-1-isocyanato-ethyl)-benzol bei einer Temperatur von 180° C und unter einem Druck von 1 mbar abdestilliert.

Man erhielt in drei verschiedenen Ansätzen jeweils 570 Gew.-Teile einer Mischung von Carbodiimiden mit einem NCO-Gehalt von

| | |
|---|---|
| CDI 1 | 8,0 % |
| CDI 2 | 10,9 % und |
| CDI 3 | 7,9 %, |

die einen Gehalt an -N=C=N--Gruppen von ca. 15 Gew.-% (berechnet), einen Schmelzpunkt < 30°C und eine Iodfarbzahl von 5 bis 7 gemessen nach DIN 6162 aufwiesen..

A) Herstellung der Carbodiimide

Anschließend wurden die Carbodiimide (CDI) 1, 2 oder 3 in einem Rührkolben mit den in der Tabelle 1 angegebenen Alkoholen bei 140°C umgesetzt, bis ein NCO-Gehalt < 0,2 Gew.-% erreicht war.

Als Verbindung (i) wurde ein Methoxypolyoxyethylenalkohol mit einem zahlenmittleren Molgewicht von 520 g/mol und einem Gehalt an EO-Einheiten von 94,0 Gew.-% verwendet.

Die verwendeten Verbindungen (ii) sind in der Tabelle 1 angegeben.

Die Ansätze wurden so berechnet, daß stets $\Sigma(A+B)/NCO = 1$ galt.

Tabelle 1:

| Beispiel | NCO-Gehalt Carbodiimid | Masse MPG | Molzahl MPG | B | Masse B | Molzahl B | Gehalt EO aus MPG |
|---|---|---|---|---|---|---|---|
| | (Gew.%) | (g) | (mol) | | (g) | (mol) | (Gew.%) |
| 1 (Vergleich) | 8,0 | ./. | ./. | DEGMBE | 154,5 | 0,952 | ./. |
| 2 (Vergleich) | 8,0 | ./. | ./. | DEGMEE | 127,8 | 0,952 | ./. |
| 3 (Vergleich) | 8,0 | 495,2 | 0,952 | ./. | ./. | ./. | 46,8 |
| 4 | 8,0 | 240,6 | 0,476 | EH | 62,0 | 0,476 | 28,2 |
| 5 | 8,0 | 165,1 | 0,317 | EH | 82,7 | 0,635 | 21,2 |
| 6 | 8,0 | 123,8 | 0,238 | EH | 93,0 | 0,714 | 16,6 |
| 7 | 10,9 | 337,4 | 0,649 | DEGMEE | 87,1 | 0,649 | 35,0 |
| 8 | 10,9 | 224,9 | 0,433 | DEGMEE | 116,1 | 0,865 | 25,7 |
| 9 | 10,9 | 168,7 | 0,325 | DEGMEE | 130,6 | 0,974 | 20,3 |
| 10 | 10,9 | 126,4 | 0,243 | DEGMEE | 141,4 | 1,054 | 15,8 |
| 11 | 10,9 | 94,3 | 0,181 | DEGMEE | 149,8 | 1,116 | 12,2 |
| 12 | 7,9 | 240,8 | 0,463 | DEGMEE | 64,1 | 0,477 | 28,7 |
| 13 | 7,9 | 190,3 | 0,366 | DEGMEE | 77,8 | 0,574 | 23,8 |
| 14 | 7,9 | 171,6 | 0,330 | DEGMEE | 81,9 | 0,610 | 21,9 |
| 15 | 7,9 | 208,0 | 0,400 | DEGMEE | 72,5 | 0,540 | 25,6 |

DEGMBE: Diethylengleykolmonobutylether, DEGMEE: Diethylenglykolmonoethylether, EH: 2-Ethylhexanol

B) Herstellung von PUR-Elastomeren

Zur Herstellung von PUR-Elastomeren wurde ein Prepolymer aus 1000 Gewichtsteilen Ethylenglykoladipat (Hydroxylzahl 56) und 380 g 4,4'-MDI hergestellt.

Aus den oben beschriebenen Carbodiimiden und Ethoxyalaten der Rizinol- und Ölsäure als Schaumstabilisatoren wurde die Vernetzerkomponente mit einem Wassergehalt von 37,3 % hergestellt.

Die Carbodiimidmenge wurde so gewählt, daß in fertigen PU-Elastomeren ein Anteil von 0,8 Gew.-% des jeweiligen Carbodiimides erhalten wurde.

Die so hergestellte Vernetzerkomponente wurde unter kräftigem Rühren zu dem auf 90°C abgekühlten Prepolymer gegeben. Das Äquivalenzverhältnis NCO/OH betrug dabei 1,07.

Nach einer Rührzeit von insgesamt 8 Sek. wurde die Reaktionsmischung in eine auf 90°C erhitzte verschließbare Form gefüllt und 25 Min. ausgehärtet. Die Form war so gestaltet, daß eine zylindrische Prüffeder mit drei Segmenteinschnürungen und einer Höhe von 100 mm, einem Außendurchmesser von 50 mm und einem Innendurchmesser von 10 mm erhalten wurde. Nach Entformung wurde die Feder einer thermischen Nachhärtung bei 110°C über 16 h unterzogen.

Zur Prüfung der dynamisch-mechanischen Eigenschaften wurden die Federn 100.000 Lastwechseln mit einer Kraft von 6kN und einer Frequenz von 1,2 Hz ausgesetzt. Der Setzbetrag SB wird ermittelt nach der Gleichung

$$SB = (H_o - H_r) * 100/H_o$$

$H_o$ ist die Höhe der Feder vor Beginn der Prüfung; $H_r$ ist die Höhe der Feder nach der Prüfung, gemessen nach 24 Stunden Lagerung bei 23°C/50 % rel. Feuchte.

Die Federn wurden visuell auf ihren Zustand nach der Prüfung untersucht. Die Angabe "nach <Zahl> ex", bedeutet, daß die Feder nach <Zahl> Lastwechseln zerstört war. "i.O." bedeutet "in Ordnung". Es wurden stets zwei Federn/Rezept geprüft.

Die gemessenen Eigenschaften sind in der Tabelle 2 dargestellt.

Tabelle 2:

| Beispiel | Carbodiimid aus Beispiel | Setzbetrag | Zustand der Federn nach 100.000 Lastwechseln |
|---|---|---|---|
| 16 a | 3 (Vergleich) | ./. | nach 50.000 ex |
| 16 b | 3 (Vergleich) | 24,1 | Riß |
| 17 a | 1 (Vergleich) | ./. | nach 79.000 ex |
| 17 b | 1 (Vergleich) | 15,0 | Riß |
| 18 a | 2 (Vergleich) | ./. | nach 45.000 ex |
| 18 b | 2 (vergleich) | ./. | nach 55.000 ex |
| 19 a | 4 | 14,8 | i.o. |
| 19 b | 4 | 18,3 | Riß |
| 20 a | 5 | 18,3 | i.o. |
| 20 b | 5 | 13,2 | i.o. |
| 21 a | 6 | 15,1 | i.o. |
| 21 b | 6 | 15,5 | i.o. |
| 22 a | 7 | 14,9 | i.o. |
| 22 b | 7 | 14,7 | i.o. |
| 23 a | 8 | 14,9 | i.o. |
| 23 b | 8 | 15,9 | i.o. |
| 24 a | 9 | 12,3 | i.o. |
| 24 b | 9 | 12,9 | i.o. |
| 25 a | 10 | 15,3 | i.o. |
| 25 b | 10 | 17,3 | i.o. |
| 26 a | 11 | 16,9 | i.o. |

Tabelle 2: (fortgesetzt)

| Beispiel | Carbodiimid aus Beispiel | Setzbetrag | Zustand der Federn nach 100.000 Lastwechseln |
|---|---|---|---|
| 26 b | 11 | 16,6 | i.o. |
| 27 a | 12 | 17,6 | i.o. |
| 27 b | 12 | 18,2 | i.o. |
| 28 a | 13 | 12,5 | i.o. |
| 28 b | 13 | 13,0 | i.o. |
| 29 a | 14 | 12,8 | i.o. |
| 29 b | 14 | 12,8 | i.o. |
| 30 a | 15 | 14,1 | i.o. |
| 30 b | 15 | 13,8 | i.o. |
| 31 a | 16 | 13,7 | i.o. |
| 31 b | 16 | 14,0 | i.o. |

Anhand dieser Meßergebnisse wird deutlich, daß die erfindungsgemäßen Carbodiimide insbesondere bei Einsatz als Stabilisatoren in Polyurethanelastomeren zu Produkten mit deutlich verbesserten Eigenschaften führen.

**Patentansprüche**

1. Carbodiimide auf der Basis von 1,3-Bis-(1-methyl-1-isocyanato-ethyl)-benzol enthaltend 12 bis 40 Gew.-% Ethylenoxideinheiten [-$CH_2$-$CH_2$-O-], bezogen auf das Gewicht der Carbodiimide.

2. Verfahren zur Herstellung von Carbodiimiden auf der Basis von 1,3-Bis-(1-methyl-1-isocyanato-ethyl)-benzol enthaltend 12 bis 40 Gew.-% Ethylenoxideinheiten [-$CH_2$-$CH_2$-O-], bezogen auf das Gewicht der Carbodiimide, **dadurch gekennzeichnet, daß** man 1,3-Bis-(1-methyl-1-isocyanato-ethyl)-benzol in Gegenwart von Katalysatoren unter Kohlendioxidabspaltung zu Carbodiimiden umsetzt und anschließend das Isocyanatgruppen-aufweisende Carbodiimid mit einer gegenüber Isocyanaten reaktiven Verbindung (i) umsetzt, die Ethylenoxideinheiten [-$CH_2$-$CH_2$-O-] aufweist und wobei man als gegenüber Isocyanaten reaktive Verbindungen zur Herstellung der Carbodiimide eine Mischung einsetzt, die enthält:

   (i) gegenüber Isocyanaten reaktive Verbindungen, die mindestens 5 Ethylenoxideinheiten aufweisen und
   (ii) gegenüber Isocyanaten reaktive Verbindungen, die keine oder weniger als 5 Ethylenoxideinheiten aufweisen.

3. Verfahren zur Herstellung von Carbodiimiden auf der Basis von 1,3-Bis-(1-methyl-1-isocyanato-ethyl)-benzol enthaltend 12 bis 40 Gew.-% Ethylenoxideinheiten [-$CH_2$-$CH_2$-O-], bezogen auf das Gewicht der Carbodiimide, **dadurch gekennzeichnet daß** man 1,3-Bis-(1-methyl-1-isocyanato-ethyl)-benzol mit mindestens einer Verbindung (i) umsetzt, die Ethylenoxideinheiten [-$CH_2$-$CH_2$-O-] aufweist, wobei das Verhältnis von eingesetzten Isocyanatgruppen zu gegenüber Isocyanaten reaktiven Gruppen mindestens 2 : 1 beträgt, und anschließend das Isocyanatgruppen aufweisende Reaktionsprodukt in Gegenwart von Katalysatoren unter Kohlendioxidfreisetzung zu Carbodiimiden umsetzt und wobei man als gegenüber Isocyanaten reaktive Verbindungen zur Herstellung der Carbodiimide eine Mischung einsetzt, die enthält:

   (i) gegenüber Isocyanaten reaktive Verbindungen, die mindestens 5 Ethylenoxideinheiten aufweisen und
   (ii) gegenüber Isocyanaten reaktive Verbindungen, die keine oder weniger als 5 Ethylenoxideinheiten aufweisen.

4. Carbodiimide erhältlich durch ein Verfahren gemäß einem der Ansprüche 2 und 3.

5. Mischungen enthaltend Verbindungen, die Esterstrukturen aufweisen, und Carbodiimide gemäß Anspruch 1.

6. Mischungen enthaltend Carbodiimide gemäß Anspruch 1 und. mindestens eine Verbindung aus der folgenden Gruppe: Polyurethane, die Esterstrukturen aufweisen, Polykondensationsprodukte wie thermoplastische Polyester

wie Polyethylen- und -butylenterephthalat, Polyetherester, Polyesterester, Polyesteramide, Polycaprolactone, ungesättigte Polyesterharze und Polyamide.

**7.** Mischungen gemäß Anspruch 5 oder 6 enthaltend Carbodiimidegemäß Anspruch 1 in einer Menge von 0,05 bis 10 Gew.-%, bezogen auf das Gesamtgewicht der Mischung.

**8.** Verwendung von Carbondiimiden gemäß Anspruch 1 als Stabilisatoren gegen den hydrolytischen Abbau von Estergruppen enthaltenden Verbindungen.

**9.** Verfahren zur Herstellung von Polyurethanen durch Umsetzung von Isocyanaten, gegenüber Isocyanaten reaktiven Verbindungen, Treibmitteln und gegebenenfalls Katalysatoren, Hilfs- und/oder Zusatzstoffen, **dadurch gekennzeichnet, daß** die Umsetzung in Gegenwart von Carbodiimiden gemäß Anspruch 1 erfolgt.

**10.** Polyurethane erhältlich durch ein Verfahren gemäß Anspruch 9.

**11.** Verwendung von Carbodiimiden gemäß Anspruch 1 als Vernetzer für wäßrige Latices.

## Claims

**1.** A carbodiimide based on 1,3-bis(1-methyl-1-isocyanatoethyl)benzene containing from 12 to 40% by weight of ethylene oxide units [$-CH_2-CH_2-O-$], based on the weight of the carbodiimide.

**2.** A process for preparing carbodiimides based on 1,3-bis(1-methyl-1-isocyanatoethyl)benzene containing from 12 to 40% by weight of ethylene oxide units [$-CH_2-CH_2-O-$], based on the weight of the carbodiimides, which comprises reacting 1,3-bis(1-methyl-1-isocyanatoethyl)benzene in the presence of catalysts with elimination of carbon dioxide to form carbodiimides and subsequently reacting the carbodiimide containing isocyanate groups with a compound (i) which is reactive toward isocyanates and comprises ethylene oxide units [$-CH_2-CH_2-O-$], where the isocyanate-reactive compounds used for preparing the carbodiimides are mixtures comprising:

(i) compounds which are reactive toward isocyanates and contain at least 5 ethylene oxide units and
(ii) compounds which are reactive toward isocyanates and contain no or less than 5 ethylene oxide units.

**3.** A process for preparing carbodiimides based on 1,3-bis(1-methyl-1-isocyanatoethyl(benzene containing from 12 to 40% by weight of ethylene oxide units [$-CH_2-CH_2-O-$], based on the weight of the carbodiimides, which comprises reacting 1,3-bis(1-methyl-1-isocyanatoethyl)benzene with at least one compound (i) comprising ethylene oxide units [$-CH_2-CH_2-O-$], where the ratio of isocyanate groups used to groups which are reactive toward isocyanates is at least 2:1, and subsequently reacting the reaction product containing isocyanate groups in the presence of catalysts with elimination of carbon dioxide to form carbodiimides, where the isocyanate-reactive compounds used for preparing the carbodiimides are mixtures comprising:

(i) compounds which are reactive toward isocyanates and contain at least 5 ethylene oxide units and
(ii) compounds which are reactive toward isocyanates and contain no or less than 5 ethylene oxide units.

**4.** A carbodiimide obtainable by a process as claimed in claim 2 or 3.

**5.** A mixture comprising compounds which contain ester structures and carbodiimides as claimed in claim 1.

**6.** A mixture comprising carbodiimides as claimed in claim 1 and at least one compound selected from the following group: polyurethanes containing ester structures, polycondensation products such as thermoplastic polyesters such as polyethylene terephthalate and polybutylene terephthalate, polyether esters, polyester esters, polyesteramides, polycaprolactones, unsaturated polyester resins and polyamides.

**7.** A mixture as claimed in claim 5 or 6 comprising carbodiimides as claimed in claim 1 in an amount of from 0.05 to 10% by weight, based on the total weight of the mixture.

**8.** The use of carbodiimides as claimed in claim 1 as stabilizers against hydrolytic degradation of compounds containing ester groups.

9. A process for preparing polyurethanes by reaction of isocyanates, compounds which are reactive toward isocyanates, blowing agents and, if desired, catalysts, auxiliaries and/or additives, wherein the reaction is carried out in the presence of carbodiimides as claimed in claim 1.

10. A polyurethane obtainable by a process as claimed in claim 9.

11. The use of carbodiimides as claimed in claim 1 as crosslinkers for aqueous latices.

## Revendications

1. Carbodiimides à base du 1,3-bis-(1-méthyl-1-isocyanato-éthyl)-benzène contenant des unités d'oxyde d'éthylène [-$CH_2$-$CH_2$-O-] à concurrence de 12 à 40 % en poids, rapportés au poids des carbodiimides.

2. Procédé pour la préparation de carbodiimides à base du 1,3-bis-(1-méthyl-1-isocyanato-éthyl)-benzène contenant des unités d'oxyde d'éthylène [-$CH_2$-$CH_2$-O-] à concurrence de 12 à 40 % en poids, rapportés au poids des carbodiimides, **caractérisé en ce qu'**on fait réagir du 1,3-bis-(1-méthyl-1-isocyanato-éthyl)-benzène en présence de catalyseurs avec élimination du dioxyde de carbone pour obtenir des carbodiimides et on fait ensuite réagir le carbodiimide présentant des groupes isocyanates avec un composé (i) réactif vis-à-vis de groupes isocyanates, qui présente des unités d'oxyde d'éthylène [-$CH_2$-$CH_2$-O-], et dans lequel on met en oeuvre, à titre de composé réactif vis-à-vis de groupes isocyanates, pour la préparation des carbodiimides, un mélange qui contient :

   (i) des composés réactifs vis-à-vis de groupes isocyanates, qui présentent au moins 5 unités d'oxyde d'éthylène et
   (ii) des composés réactifs vis-à-vis de groupes isocyanates qui ne présentent aucune unité d'oxyde d'éthylène ou qui présentent moins de 5 unités d'oxyde d'éthylène.

3. Procédé pour la préparation de carbodiimides à base du 1,3-bis-(1-méthyl-1-isocyanato-éthyl)-benzène contenant des unités d'oxyde d'éthylène [-$CH_2$-$CH_2$-O-] à concurrence de 12 à 40 % en poids, rapportés au poids des carbodiimides, **caractérisé en ce qu'**on fait réagir du 1,3-bis-(1-méthyl-1-isocyanato-éthyl)-benzène avec au moins un composé (i) qui présente des unités d'oxyde d'éthylène [-$CH_2$-$CH_2$-O-], le rapport des groupes isocyanates mis en oeuvre aux groupes réactifs vis-à-vis de groupes isocyanates s'élevant à au moins 2 : 1, et on fait ensuite réagir le produit réactionnel présentant des groupes isocyanates en présence de catalyseurs avec libération de dioxyde de carbone pour obtenir des carbodiimides, et dans lequel on met en oeuvre, à titre de composé réactif vis-à-vis de groupes isocyanates, pour la préparation des carbodiimides, un mélange qui contient :

   (i) des composés réactifs vis-à-vis de groupes isocyanates, qui présentent au moins 5 unités d'oxyde d'éthylène et
   (ii) des composés réactifs vis-à-vis de groupes isocyanates qui ne présentent aucune unité d'oxyde d'éthylène ou qui présentent moins de 5 unités d'oxyde d'éthylène.

4. Carbodiimides que l'on obtient via un procédé selon l'une quelconque des revendications 2 et 3.

5. Mélanges contenant des composés qui présentent des structures esters et des carbodiimides selon la revendication 1.

6. Mélanges contenant des carbodiimides selon la revendication 1 et au moins un composé choisi parmi le groupe ci-après : des polyuréthanes qui présentent des structures esters ; des produits de polycondensation tels que des polyesters thermoplastiques comme le polyéthylène téréphtalate et le polybutylène téréphtalate, des polyétheresters, des polyester-esters, des polyesteramides, des polycaprolactones, des résines de polyesters insaturées et des polyamides.

7. Mélanges selon la revendication 5 ou 6 contenant des carbodiimides selon la revendication 1 en une quantité de 0,05 à 10 % en poids, rapportés au poids total du mélange.

8. Utilisation de carbodiimides selon la revendication 1, à titre de stabilisateurs contre la dégradation hydrolytique de composés contenant des groupes esters.

9. Procédé pour la préparation de polyuréthanes par mise en réaction d'isocyanates, de composés réactifs vis-à-vis de groupes isocyanates, d'agents moussants et le cas échéant de catalyseurs, d'adjuvants et/ou d'additifs, **caractérisé en ce que** la mise en réaction a lieu en présence de carbodiimides selon la revendication 1.

10. Polyuréthanes que l'on obtient via un procédé selon la revendication 9.

11. Utilisation de carbodiimides selon revendication 1 à titre d'agents de réticulation pour des latex aqueux.